# EUROPEAN PATENT SPECIFICATION

(11) **EP 0 441 328 B1**
(45) Date of publication and mention of the grant of the patent: **05.10.1994**
(21) Application number: 91101543.6
(22) Date of filing: 05.02.1991
(51) Int. Cl.: G01F 1/84

(54) **Perfected differential flowmeter**
Differential-Durchflussmesser
Débitmètre différentiel

(30) Priority: 08.02.1990 IT 472190 U
(43) Date of publication of application: 14.08.1991
(73) Proprietor: BELLCO S.p.A., I-41037 Mirandola (IT)
(72) Inventor: Cianciavicchia,Domenico, I-64100-Teramo-Cavuccio (IT); Facchini,Mauro, I-41037 Mirandola (IT); Morselli,Massimo, I-41038 S.Felice sul panaro (IT); Pradelli,Alessandro, I-41034 Finale Emilia (IT)
(74) Representative: Prato, Roberto

(56) References cited:
- EP-A- 0 244 692
- US-A- 4 252 028
- US-A- 4 911 006

## Description

The present invention relates to a differential flowmeter for determining the difference in mass flow (grams/minutes) along two hydraulic circuits, and may be used on a dialysis unit for measuring the difference in mass flow of the dialysis fluid up- and downstream from the filter, and so determining the amount of ultrafiltered fluid and, therefore, water and electrolytes transferred from the blood to the dialysis fluid. Such a flowmeter is disclosed in EP-A-0 244 692.

Current flowmeters comprise two small-diameter tubes along which a fluid flows upstream. Both tubes are the same, of the same length, are arranged side by side in the same plane and, being U-shaped, present a respective central portion from which two arms extend perpendicularly. The central portions are crossed in the middle, with a first arm of a first tube inside and parallel to a first arm of the second tube, and a second arm of the first tube outside and parallel to a second arm of the second tube. The central portions of the tubes are welded together, and the tubes welded to a base in which they are connected hydraulically to the circuits. By means of an electromagnet acting on the crossover portion, the tube assembly is oscillated, about an axis defined by the couplings between the tubes and the base, at the resonance frequency of the assembly. If flow is the same along both tubes, the central portions oscillate in the same plane. Conversely, in accordance with the Coriolis principle, the effect of the so-called Coriolis forces tilts the central portions in relation to said plane, and by an angle proportional to the difference in flow. The flowmeter is also provided with electric means for producing an electric signal proportional to said tilt angle.

Flowmeters of the aforementioned type present several drawbacks. In particular, the twin-tube system is an unstable structure which, when operated, is also subject to lateral stress in the same plane as the system. This therefore tends to oscillate in this plane also, thus resulting in inaccuracy and difficulty in determining the difference in mass flow of the two circuits. Moreover, the couplings connecting the tubes to the base are too close together, which, in addition to requiring complex, manual welding, thus increasing manufacturing time and cost, also makes hydraulic connection difficult. At present, in fact, said connections are defined by channels formed in the base and which communicate hydraulically with the tubes at the top. The channels inside the base diverge so as to allow sufficient clearance between the inlets at the bottom to install commercial couplings for connecting the circuits. Such a system, however, obviously complicates manufacture of the base.

The aim of the present invention is to provide a differential flowmeter designed to overcome the aforementioned drawbacks, i.e. which presents a structure capable of withstanding lateral stress, and provides for troublefree connection of the tubes to both the base and hydraulic circuits.

With this aim in view, according to the present invention, there is provided a differential flowmeter comprising:
a first and second substantially U-shaped tube of the same design and length, defined in the same plane, and along which a respective fluid flows upstream;
a base body to which the ends of said tubes are connected;
first means for oscillating said tubes at the resonance frequency of the system defined by the same and about an axis defined by the connecting points of said tubes to said body; and
second means for detecting the tilt angle assumed during said oscillation by integral, crossed central portions of said tubes, opposite said body and defined by two respective parallel portions, one at a different level from the second respective portion of said central portion;
characterised by the fact that, from each said central portion, there originate two straight lateral arms integral with said body, said arms of said first tube respectively defining an obtuse and an acute angle with said central portion, and said arms of said second tube respectively defining an acute and an obtuse angle with said central portion.

A preferred, non-limiting embodiment of the present invention will be described by way of example with reference to the accompanying drawings, in which:
Fig.1 shows a partially-sectioned front view of a differential flowmeter;
Fig.2 shows a partial side view of the Fig.1 flowmeter;
Fig.3 shows a block diagram of one application of the Fig.1 flowmeter.

Number 1 in Fig.s 1 and 2 indicates a differential flowmeter comprising two tubes 2 and 3, along which two fluids Φ1 and Φ2 flow upstream, and having means for detecting the difference in mass flow (grams/minutes) of fluids Φ1 and Φ2. Tubes 2 and 3 are of small diameter, substantially in the form of a downturned U, and connected integral with a base body 4 in which are formed connections connecting tubes 2 and 3 to respective hydraulic circuits. Tubes 2 and 3 are defined in the same plane, are the same length, and present the same flow direction. Tube 2 presents a central portion 5 parallel to the longitudinal axis of body 4 and defined by two straight parallel portions 5a and 5b of the same length but at a different distance from body 4. In particular, portion 5a is located higher than portion 5b. Tube 3 presents a central portion 6 parallel to central portion 5 and also defined by two straight parallel portions 6a and 6b of the same length but at a different distance from body 4. Central portions 5 and 6 are the same length. Portion 6a is located lower than portion 6b and in particular immediately below portion 5a and coaxial with portion 5b. Portion 6b is located over portion 5b and coaxial with portion 5a. Central portions 5 and 6 are thus crossed in the middle, at which point they are welded together.

From each central portion 5 and 6, there originate two straight lateral arms welded integral with body 4. Arms 7 and 8 originate respectively from portions 5a and 6a, and arms 11 and 12 from portions 5b and 6b. Arm 7 defines an obtuse angle with portion 5a, while arm 8 defines an acute angle with portion 6a. Said acute angle is supplementary to said obtuse angle, i.e. making a sum of 180°. Arm 11 defines with portion 5b the same acute angle defined between arm 8 and portion 6a, while arm 12 defines with portion 6b the same obtuse angle defined between arm 7 and portion 5a. This therefore defines two identical isosceles triangles, one formed by arms 7 and 8 and body 4, and the other by arms 11 and 12 and body 4.

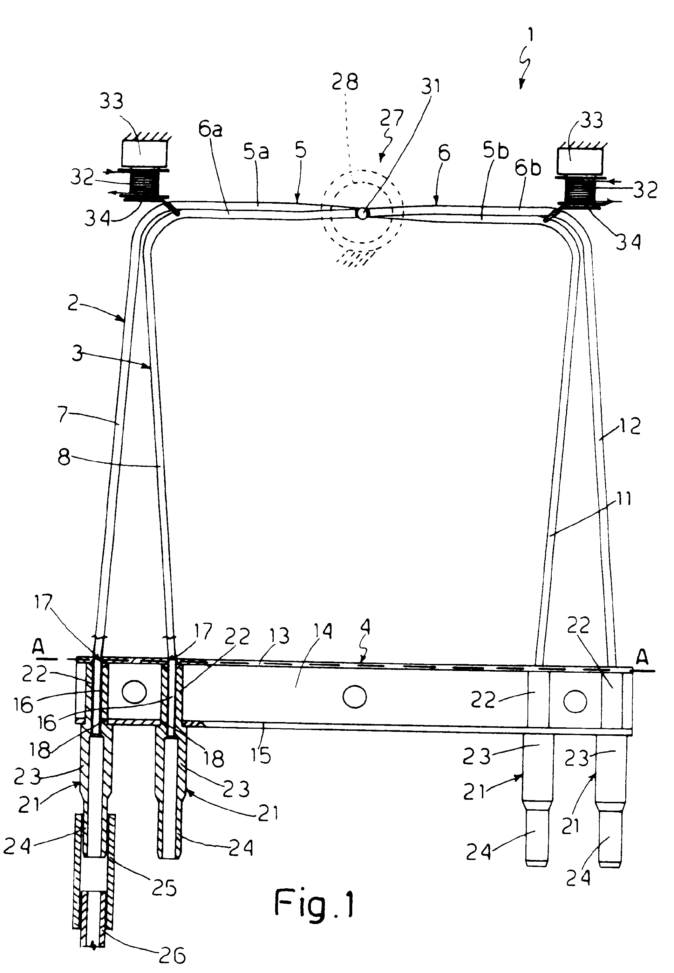

As shown in Fig.s 1 and 2, body 4 presents a C-shaped cross section defined by a top wall 13, a central wall 14 and a bottom wall 15 parallel to wall 13. Respective straight end portions 16 of arms 7, 8, 11 and 12 fit perpendicularly inside respective through holes 17 in top wall 13, at which holes 17 arms 7, 8, 11 and 12 change direction and are secured to wall 13 by means of a respective circular weld. Portions 16 extend beyond wall 15 through respective holes 18 formed in wall 15 and coaxial with holes 17. Each portion 16 is housed entirely inside a cylindrical hydraulic endpiece 21 having a first portion 22 engaged internally by portion 16 and extending inside body 4 through hole 18; a second portion 23 outside wall 15 and having larger inside and outside diameters than portion 22; and a third portion 24 having a smaller outside diameter than portion 23. By means of a hydraulic coupling 25, portion 24 provides for connecting the conduit 26 of a hydraulic circuit as shown schematically in Fig.1.

Flowmeter 1 comprises an electrical device for oscillating the system defined by tubes 2 and 3 at its resonance frequency and about an axis A-A defined by joining the connecting points of arms 7, 8, 11 and 12 to wall 13, which arms 7, 8, 11 and 12, it should be remembered, all lie in the same plane. In the embodiment shown, said electrical device consists of an electromagnet comprising an electrical coil 28, and a central permanent magnet 31 in the form of a cylindrical bar welded to the crossover point of central portions 5 and 6. Geometrically, magnet 31 presents its longitudinal axis perpendicular to central portions 5 and 6 and parallel to wall 13. In a manner not shown, coil 28 is bracketed integral with a fixed body.

Flowmeter 1 presents a second electrical device for generating an electric signal proportional to the tilt angle of central portions 5 and 6 in relation to an axis defined by the same and relative to oscillation of tubes 2 and 3 when these contain the same mass flow, as described in more detail later on. In the embodiment shown, said second device consist of two electrical coils 32, each connected to a permanent magnet 33. In particular, flowmeter 1 comprises two brackets 34, one welded to portions 5a and 6a at a first end of portions 5 and 6, and the other welded to portions 5b and 6b at a second opposite end of portions 5 and 6. Each bracket 34 supports one of coils 32, which thus oscillate together with the system. In a manner not shown, magnets 33 are bracketed integral with a fixed body.

In actual use, respective fluids are circulated upstream in tubes 2 and 3 and, by virtue of the elasticity of the system, this is oscillated about axis A-A by electromagnet 27 at the resonance frequency of the system. When so oscillated, and with the same mass flow in both tubes 2 and 3, portions 5 and 6 maintain a constant plane, i.e. oscillate parallel to axis A-A. In the presence of different mass flows in tubes 2 and 3, on the other hand, the Coriolis forces tilt portions 5 and 6 in relation to said constant plane, the tilt direction varying according to the direction of oscillation, as shown in Fig.s 3 and 4 of Italian Patent Application n.20312 A/86 filed by the present Applicant on 6 May 1986 (see also EP-A-0 244 692), and the content of which is incorporated herein by way of reference as required. As said tilt angle is proportional to the difference in mass flow, the latter can be measured by simply determining said angle. As the system is oscillated, the electrical device defined by coils 32 and magnets 33 produces an electric signal proportional to the tilt angle of portions 5 and 6 and, therefore, to the difference in mass flow circulating upstream in tubes 2 and 3. Said signal may be processed by an electronic control system for controlling, via actuators, operation of the hydraulic circuits fitted with flowmeter 1.

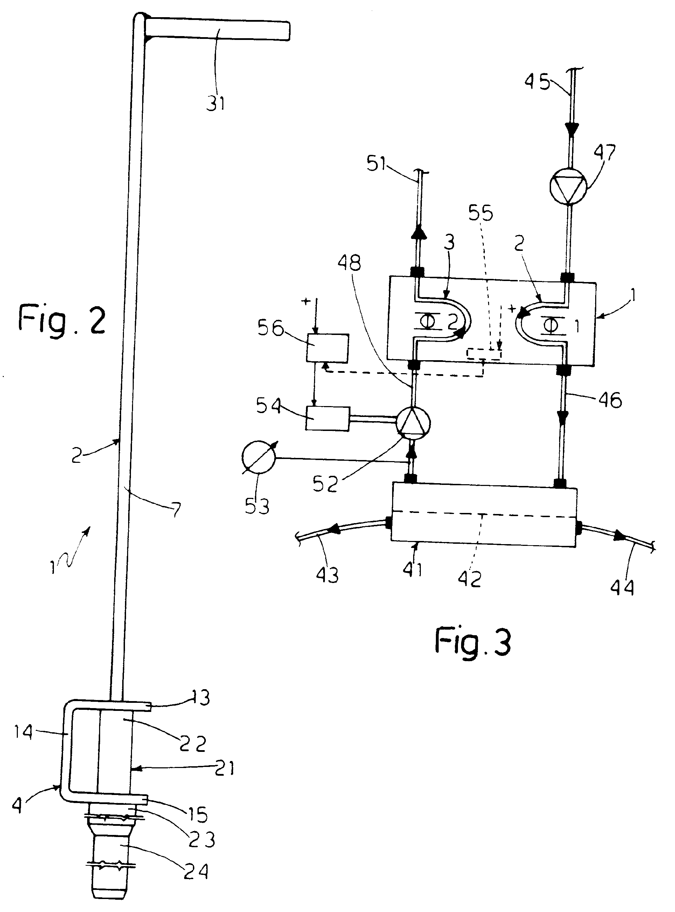

One application of flowmeter 1 will be described by way of example with reference to Fig.3, which shows a block diagram of part of a dialysis unit in which flowmeter 1 is used for measuring ultrafiltration.

Said unit comprises:
a known dialyzer 41 having a membrane 42 separating a first circuit, defined by an input conduit 43 and an output conduit 44 along which the patient's blood is circulated, from a second circuit along which is circulated the dialysis fluid;
a conduit 45 along which the fresh dialysis fluid is circulated and which is connected to a first end of tube 2;
a conduit 46 connecting the second end of tube 2 to the inlet of said second circuit in dialyzer 41;
a first pump 47 on conduit 45;
a conduit 48 connecting the outlet of said second circuit to a first end of tube 3;
a conduit 51 connecting the second end of tube 3 to a drain (not shown);
a second pump 52 on conduit 48;
a device 53 for measuring the pressure along conduit 48 upstream from pump 52;
an electric motor 54 powering pump 52;
an electric block 55 indicating both electromagnet 27 and said device (coils 32 and magnets 33) for generating said electric signal proportional to the difference in mass flow of fluids Φ1 and Φ2; and
an electronic control system 56 for processing said signal generated by block 55 and controlling pump 52 via motor 54.

Tube 2 carries the fresh dialysis fluid and tube 3 the contaminated fluid, i.e. the fresh dialysis fluid plus the metabolic waste extracted from the blood via membrane 42. Flowmeter 1 is thus able to detect, instant by instant, the amount of waste extracted and, therefore, the degree of filtration of the blood. On the basis of the electric signal supplied by block 55 (and possibly also an electric signal indicating the pressure upstream from pump 52), control system 56 provides for controlling operation of pump 52 and possibly also pump 47.

The advantages of the present invention will be clear from the foregoing description.

Firstly, the design of tubes 2 and 3, i.e. the oblique arrangement of arms 7, 8, 11 and 12 in relation to body 4, enables the system to effectively withstand lateral stress, by virtue of the arms of one tube buttressing the other. Which advantage is achieved with no impairment in the elasticity of the system in terms of oscillation. As compared with known flowmeters, flowmeter 1 therefore provides for more accurate differential mass flow measurement. The diverging arrangement of arms 7,8 and 11,12, as shown in Fig.1, also provides for precision circular welding (even automatically) of arms 7, 8, 11 and 12 inside respective holes 17 in wall 13, as well as for inserting end portions 16 directly inside couplings (endpieces 21) for connection to conduits 26. As a result, body 4 and flowmeter 1 as a whole are both straightforward and relatively cheap to produce. The above advantages, i.e. greater precision and straightforward manufacture and installation, also make it more convenient to employ one differential flowmeter as opposed to two flowmeters, one for each fluid, thus overcoming the drawbacks typically associated with the use of two instruments, i.e. large size; greater number of components; setting requirements; drift caused by two electronic circuits; interference caused by differences in the resonance frequencies of the two flowmeters, etc. Finally, the advantages of flowmeter 1 are obvious in the case of a dialysis unit requiring extremely accurate differential mass flow measurements.

Flowmeter 1 may, of course, be applied to any system or unit requiring such measurements.

## Claims

1. A differential flowmeter comprising:
a first (2) and second (3) substantially U-shaped tube of the same design and length, defined in the same plane, and along which a respective fluid (Φ1, Φ2) flows upstream;
a base body (4) to which the ends of said tubes (2, 3) are connected;
first means (27) for oscillating said tubes (2, 3) at the resonance frequency of the system defined by the same and about an axis (A-A) defined by the connecting points of said tubes (2, 3) to said body (4); and
second means (32, 33) for detecting the tilt angle assumed during said oscillation by integral, crossed central portions (5, 6) of said tubes (2, 3), opposite said body (4) and defined by two respective parallel portions (5a, 5b, and 6a, 6b), one at a different level from the second respective portion of said central portion (5 or 6);
characterised by the fact that, from each said central portion (5, 6), there originate two straight lateral arms (7, 11 and 8, 12) integral with said body (4), said arms (7, 11) of said first tube (2) respectively defining an obtuse (7, 5a) and an acute angle (11, 5b) with said central portion (5), and said arms (8, 12) of said second tube (3) respectively defining an acute (8, 6a) and an obtuse (12, 6b) angle with said central portion (6).

2. A flowmeter as claimed in Claim 1, characterised by the fact that said tubes (2, 3) define the same obtuse and acute angles.

3. A flowmeter as claimed in Claim 2, characterised by the fact that the obtuse and acute angles of said tubes (2, 3) are supplementary, i.e. make a total of 180°.

4. A flowmeter as claimed in at least one of the foregoing Claims, characterised by the fact that said arms present an end portion (16) fitted inside a respective hydraulic coupling (21) for connection to a respective conduit (26).

5. A flowmeter as claimed in Claim 4, characterised by the fact that said end portions (16) are straight and perpendicular to the longitudinal axis of said body (4).

6. A flowmeter as claimed in Claim 4 and/or 5, characterised by the fact that said body (4) presents a top wall (13) having respective through holes (17) for said arms (7, 8, 11, 12), at which holes (17) said arms (7, 8, 11, 12) are secured integral with said top wall (13) via respective circular welds.

7. A flowmeter as claimed in at least one of the foregoing Claims, characterised by the fact that said second means (32, 33) provide for generating an electric signal proportional to the detected tilt angle.

8. A dialysis unit, characterised by the fact that it comprises:
a differential flowmeter (1) as claimed in the foregoing Claims;
a dialyzer (41) having a membrane (42) separating a first circuit, defined by an input conduit (43) and an output conduit (44) along which the patient's blood is circulated, from a second circuit along which is circulated the dialysis fluid;
a first conduit (45) along which the fresh dialysis fluid is circulated and which is connected to a first end of said first tube (2);
a second conduit (46) connecting the second end of said first tube (2) to the inlet of said second circuit; a first pump (47) on said first conduit (45);
a third conduit (48) connecting the outlet of said second circuit to a first end of said second tube (3);
a fourth conduit (51) connecting the second end of said second tube (3) to a drain;
a second pump (52) on said third conduit (48);
an electric motor (54) powering said second pump (52);
an electric block (55) defined by said first means (27) and said second means (32, 33) for generating said electric signal proportional to the difference in mass flow of said fluids (Φ1, Φ2) in said tubes (2, 3); and
an electronic control system (56) for processing said signal and controlling said second pump (52) via said motor (54).

## Patentansprüche

1. Differential-Durchflußmeter mit
einem ersten Rohr (2) und einem zweiten Rohr (3), die im wesentlichen U-förmig sind und gleiche Form und Länge aufweisen, sowie in einer Ebene festgelegt sind und in denen jeweils ein Fluid (Φ1, Φ2) strömt;
einem Basiskörper (4), mit dem die Enden der Rohre (2, 3) verbunden sind;
ein erstes Mittel (27) zum Oszillieren der Rohre (2, 3) mit einer Resonanz-Frequenz des Systems, die durch das System festgelegt ist, und um eine Achse (A-A), die durch die Verbindungspunkte der Rohre (2, 3) an dem Körper (4) definiert ist, und
zweite Mittel (32, 33) zum Detektieren des Neigungswinkels, der während der Oszillation von formschlüssigen, gekreuzten Zentralabschnitten (5, 6) der Rohre (2, 3) eingenommen wird, welche dem Körper (4) gegenüberliegen und durch zwei entsprechend parallele Abschnitte (5a, 5b, und 6a, 6b) festgelegt sind, von denen einer auf einer unterschiedlichen Höhe von dem entsprechenden zweiten Abschnitt des Zentralabschnitts (5 oder 6) ist;
dadurch gekennzeichnet, daß
von jedem der Zentralabschnitte (5, 6) zwei gerade seitliche Arme (7, 11 und 8, 12) ausgehen, die mit dem Körper (4) integriert sind, wobei die Arme (7, 11) des ersten Rohrs (2) einen stumpfen Winkel (7, 5a) bzw. einen spitzen Winkel (11, 5b) mit dem Zentralabschnitt (5) bilden, und die Arme (8, 12) des zweiten Rohrs (3) entsprechend einen spitzen Winkel (8, 6a) bzw. einen stumpfen Winkel (12, 6b) mit dem Zentralabschnitt (6) bilden.

2. Durchflußmeter nach Anspruch 1, dadurch gekennzeichnet, daß die Rohre (2, 3) den gleichen stumpfen bzw. spitzen Winkel bilden.

3. Durchflußmeter nach Anspruch 2, dadurch gekennzeichnet, daß der stumpfe und der spitze Winkel der Rohre (2, 3) sich ergänzen, d.h. zu einem Gesamten von 180°.

4. Durchflußmeter nach mindestens einem der vorhergehenden Ansprüchen, dadurch gekennzeichnet, daß die Arme einen Endabschnitt (16) aufweisen, der in eine entsprechende hydraulische Kopplung (21) zur Verbindung mit einer entsprechenden Leitung (26) eingepaßt ist.

5. Durchflußmeter nach Anspruch 4, dadurch gekennzeichnet, daß die Endabschnitte (16) gerade und senkrecht zur longitudinalen Achse des Körpers (4) sind.

6. Durchflußmeter nach einem der Ansprüche 4 oder 5, dadurch gekennzeichnet, daß der Körper (4) eine obere Wand (13) aufweist, in die jeweilige Durchgangslöcher (17) für die Arme (7, 8, 11, 12) vorgesehen sind, wobei in die Löcher (17) die Arme (7, 8, 11, 12) formschlüssig mit der oberen Wand (13) mittels entsprechenden ringförmigen Verschweißungen befestigt sind.

7. Durchflußmeter nach mindestens einem der vorhergehenden Ansprüche, dadurch gekennzeichnet, daß die zweiten Mittel (32, 33) die Erzeugung eines elektrischen Signals ermöglichen, welches proportional zu dem detektierten Neigungswinkel ist.

8. Dialyseeinheit
gekennzeichnet durch
ein Differential-Durchflußmeter (1) nach einem der vorhergehenden Ansprüche;
einen Dialysator (41), der eine Membran (42) aufweist, die einen ersten Kreislauf, der durch die Eingangsleitung (43) und einer Ausgangsleitung (44) definiert ist, entlang derer das Blut eines Patienten zirkuliert wird, von einem zweiten Kreislauf, entlang dem das DialyseFluid zirkuliert wird, trennt.
eine erste Leitung (45), entlang der das frische Dialysefluid zirkuliert wird und welche mit einem ersten Ende des ersten Rohres (2) in Verbindung steht.
eine zweite Leitung (46), die das zweite Ende des ersten Rohres (2) mit dem Eingang des zweiten Kreislaufes verbindet; eine erste Pumpe (47) in der ersten Leitung (45);
eine dritte Leitung (48), die den Ausgang des zweiten Kreislaufs mit einem ersten Ende des zweiten Rohres (3) verbindet;
eine vierte Leitung (51), die das zweite Ende des zweiten Rohres (3) mit einem Abfluß verbindet.
eine zweite Pumpe (52) in der dritten Leitung (48);
einen Elektromotor (54), der die zweite Pumpe (52) betreibt;
eine Elektroeinheit (55), die durch das erste Mittel (27) und die zweiten Mittel (32, 33) festgelegt ist, zur Erzeugung des elektrischen Signals, welches proportional zu der Differenz des Massenflusses der Fluide (Φ1, Φ2) in den Rohren (2, 3) ist; und
ein elektronisches Steuerungssytem (56) zur Verarbeitung des Signals und zur Steuerung der zweiten Pumpe (52) über den Motor (54).

## Revendications

1. Débitmètre différentiel comprenant :
un premier (2) et deuxième (3) tube sensiblement en forme de U de la même structure et longueur définis dans le même plan, et dans lesquels un fluide respectif (Φ1, Φ2) s'écoule vers l'amont,
un corps de base (4) auquel les extrémités des tubes (2,3) sont connectées,
des premiers moyens (27) pour faire osciller les tubes (2,3) la fréquence de résonance du système défini par ceux-ci et autour d'un axe (A-A) défini par les points de connexion des tubes (2,3) au corps (4), et
des deuxièmes moyens (32,33) pour détecter l'angle d'inclinaison durant l'oscillation par les parties centrales intégrantes croisées (5,6) des tubes (2,3), l'opposé du corps (4) et défini par deux parties parallèles respectives (5a, 5b, et 6a, 6b), l'une à un niveau différent par rapport à la deuxième partie respective de la partie centrale (5 ou 6),
caractérisé par le fait que, de chaque partie centrale (5,6) s'étendent deux bras latéraux droits (7,11 et 8, 12) faisant partie intégrante avec le corps (4), les bras (7,11) du premier tube (2) définissant respectivement un angle obtus (7,5a) et un angle aigu (11,5b) avec la partie centrale (5), et les bras (8,12) du deuxième tube (3) définissant respectivement un angle aigu (8,6a) et un angle obtus (12,6b) avec la partie centrale (6).

2. Débitmètre selon la revendication 1, caractérisé par le fait que les tubes (2,3) définissent les mêmes angles obtus et aigu.

3. Débitmètre selon la revendication 2, caractérisé par le fait que les angles obtus et aigu des tubes (2,3) sont des angles supplémentaires, c'est-à-dire que leur somme fait 180°.

4. Débitmètre selon l'une quelconque des revendications précédentes, caractérisé par le fait que les bras présentent une partie terminale (16) placée l'intérieur d'un couplage hydraulique respectif (21) pour la connexion à une conduite respective (26).

5. Débitmètre selon la revendication 4, caractérisé par le fait que les parties terminales (16) sont droites et perpendiculaires à l'axe longitudinal du corps (4).

6. Débitmètre selon la revendication 4 et/ou 5, caractérisé par le fait que le corps (4) présente une paroi supérieure (13) ayant des ouvertures respectives (17) pour les bras (7,8,11,12), ouvertures (17) où les bras (7,8,11,12) sont fixés de façon intégrante a la paroi supérieure (13) au moyen de soudures circulaires respectives.

7. Débitmètre selon l'une des revendications précédentes, caractérisé par le fait que les deuxièmes moyens (32,33) permettent de générer un signal électrique proportionnel a l'angle d'inclinaison détecté.

8. Unité de dialyse, caractérisée par le fait qu'elle comprend :
une débitmètre différentiel (1) tel que revendiqué dans les revendications précédentes,
un dialyseur (41) comprenant une membrane (42) séparant un premier circuit défini par une conduite d'entrée (43) et une conduite de sortie (44) dans lesquelles le sang du patient circule, d'un deuxième circuit dans lequel circule le fluide de dialyse ;
une première conduite (45) dans laquelle le fluide frais de dialyse circule et qui est reliée a une première extrémité du premier tube (2) ;
une deuxième conduite (46) connectant la seconde extrémité du premier tube (2) a l'entrée du second circuit ; une première pompe (47) sur la première conduite (45) ;
une troisième conduite (48) connectant la sortie du deuxième circuit à une première extrémité du second tube (3) ;
une quatrième conduite (51) connectant la seconde extrémité du second tube (3) à un drain ;
une deuxième pompe (52) sur la troisième conduite (48) ;
un moteur électrique (54) actionnant la seconde pompe (52) ;
un bloc électrique (55) défini par les premiers moyens (27) et les seconds moyens (32,33) permettant de générer un signal électrique proportionnel a la différence de débit massique des fluides (Φ1,Φ2) dans les tubes (2,3) ; et un système de contrôle électronique (56) permettant de traiter le signal généré et de contrôler la deuxième pompe (52) via un moteur (54).
